# EUROPEAN PATENT SPECIFICATION

(11) **EP 0 532 327 B1**
(45) Date of publication and mention of the grant of the patent: **17.04.1996**
(21) Application number: 92308247.3
(22) Date of filing: 10.09.1992
(51) Int. Cl.: B01J 31/18, C07D 487/22, C07C 29/50

(54) **Cyano- and polycyanometalloporphyrins as catalysts for alkane oxidation**
Cyano- und Polycyanometalloporphyrine als Katalysatoren bei der Oxydierung von Alkanen
Cyano- et polycyanométalloporphyrines comme catalyseurs pour l'oxydation d'alcanes

(30) Priority: 12.09.1991 US 758148; 02.06.1992 US 892107
(43) Date of publication of application: 17.03.1993
(73) Proprietor: SUN COMPANY, INC. (R&M), Philadelphia, PA 19103-1699 (US)
(72) Inventor: Ellis, Paul E., Jr., Downingtown, PA 19335 (US); Lyon, James E., Wallingford, PA 19086 (US)
(74) Representative: Lewin, John Harvey

(56) References cited:
- WO-A-88/07988
- US-A- 5 004 551
- CATALYSIS LETTERS vol. 8, no. 1, January 1991, BASEL. SWITZERLAND E.LYONS, E. ELLIS 'SELECTIVE LOW TEMPERATURE HYDROXYLATION OF ISOBUTANE BY MOLECULAR OXYGEN CATALYZED BY AN IRON PERHALOPOPHYRIN COMPLEX'

## Description

### BACKGROUND OF THE INVENTION

This invention relates to oxidation of alkanes using metalloporphyrins as catalysts, and more particularly to such processes in which cyano groups have been substituted for hydrogen in the porphyrin ligand.

The use of metalloporphyrins as catalysts for the oxidation of hydrocarbons with air in the liquid phase has been shown by the inventors in U.S. Patents 4,895,680 and 4,895,682 with the further finding that halogenation of the porphyrin ring led to even more active and stable catalysts (U.S. Patents 4,900,871; 4,970,348 and U.S. Patent application Serial No. 568,118 = EP-A-0 471 561). Since these discoveries, we have been able to correlate increased electron withdrawal from halogenation of the porphyrin ring to increased catalytic air oxidation activity. Je. E. Lyons and P. E. Ellis, Jr., Catalysis Letters, 8, 45 (1991).

Other functional groups besides halogens can lead to increased electron withdrawal from the metal center in metalloporphyrins. for example, cyano groups are known for their large electron withdrawing inductive effects and cyano containing metalloporphyrins with cyano groups in the beta or pyrrolic positions have been shown to be more easily reduced than their precursors without cyano substitution. R. J. Donohoe, M. Atamian and D. F. Bocian, J. Amer. Chem. Soc., 109, 5593 (1987).

### DESCRIPTION OF THE INVENTION

We have now found that cyanometalloporphyrins and cyano/halogenometalloporphyrins have utility as catalysts for the air oxidation of alkanes such as methane, ethane, propane and butanes.

The catalysts of the invention are particularly effective in the oxidation of alkanes, and alkenes, including cycloalkanes, substituted alkanes and alkenes and the like. The starting materials thus include straight and branched-chain compounds having from 1 to 2 carbon atoms, preferably 1 to 10 carbon atoms, such as methane, ethane, propane, n-butane, isobutane, n-pentane, n-hexane, 2-methylpentane, 3-methylpentane, heptane, 2-methylheptane, 3-methylheptane and the corresponding alkene forms, as well as cycloalkanes and cycloalkenes having from 5 to 20 carbon atoms, preferably 5 to 10 carbon atoms, such as cyclopentane, cyclohexane, cycloheptane, cyclooctane and the corresponding alkene forms. These compounds, if desired, may be substituted with various moieties, although care should be taken to exclude substituents which will adversely affect the activity of the catalyst.

The oxidation, which may be carried out in a generally known manner, is desirably conducted in the liquid phase, although this is not critical, using such organic solvents as benzene, acetic acid, acetonitrile, methyl acetate, or like solvents which are inert to the conditions of the reactions, or in a neat solution of the hydrocarbon if it is liquid, and under pressures ranging from 1.03 to 103 bar (15 to 1500 psig), preferably 2.07 to 51.7 bar (30 to 750 psig), at temperature of from 25 to 250° C., more preferably 70 to 180° C. Depending upon whether the hydrocarbon to be oxidized is a solid, liquid or gas, it is dissolved in or bubbled through the solvent, together with air or oxygen, in the presence of the catalyst used according to the invention, for periods of time sufficient to yield the desired oxidation product, generally from 0.5 to 100 hours, and more preferably from 1 to 10 hours.

The choice of solvent, while not critical, can have an effect on the rates and selectivities obtained and should be carefully selected in order to optimize the desired results. For example, it has been found that solvents such as acetonitrile and acetic acid are often very effective for the oxidation of alkanes to form oxygen-containing compounds, whereas reactions carried out in solvents such as methyl acetate or benzene may occur more slowly. Thus, by routine experimentation, the optimum solvent for the particular process can be readily determined.

The ratios of the various reactants may vary widely, and are not critical. For example, the amount of catalyst employed can range from 10⁻⁶ to 10⁻³ moles per mole of hydrocarbon such as alkane, and more preferably from 10⁻⁵ to 10⁻⁴ mole of catalyst per mole of hydrocarbon, although other amounts are not precluded; while the amount of oxygen relative to the hydrocarbon starting material may also vary widely, generally 10⁻² to 10² moles of oxygen per mole of hydrocarbon. Care should be taken since some of the ratios fall within explosive limits. As a group, the catalysts are almost always soluble unless used in large excess. Thus, as a rule, the reactions are generally carried out homogeneously.

The process of the invention comprises contacting alkane with oxygen-containing gas in the presence of a metalloporphyrin in which 12.5 to 100 percent of the hydrogen atoms in the porphyrin ring have been replaced with cyano groups. Preferably, the metalloporphyrin contains as metal, iron, chromium, manganese, ruthenium, cobalt or copper.

In one embodiment, 4 to 28 percent of the hydrogen atoms in the porphyrin ring have been replaced with cyano groups and 0 to 72 percent of the hydrogen atoms in the porphyrin ring have been replaced with halogen. For example, in a porphyrin substituted with 20 fluorine atoms and 8 cyano groups, about 28 percent of the hydrogen atoms have been replaced with cyano groups and about 72 percent of the hydrogen atoms have been replaced with halogen atoms.

In one embodiment, 4 to 8 hydrogen atoms in the porphyrin ring have been substituted with cyano groups and 8 to 20 hydrogen atoms in the porphyrin ring have been substituted with halogen atoms.

In one embodiment, 1 to 8 of the pyrrolic hydrogens in the porphyrin ring have been replaced with cyano groups. In a further embodiment remaining halogens in the porphyrin ring have been replaced with halogen.

Preferably, all of the hydrogen atoms have been replaced either with cyano groups or halogen atoms, but this is not essential.

Specific catalysts useful according to the invention include cyanated meso-perfluorinatedalkylporphyrin, cyanated iron tetrakispentafluorophenylporphyrin and metallomesotetracyanoporphine.

Catalysts useful in the invention may be prepared by the following methods.

### EXAMPLE 1

Zinc (tetrakispentafluorophenyl β-octabromoporphine) prepared by the bromination of Zn(tetrakispentafluorophenylporphine) with Br₂ in CCl₄ is treated with 9 equivalents of CuCN in quinoline at reflux for several hours. After chromatography several of the bromines are replaced with CN groups giving, according to the conditions Zn(TPPF₂₀β-CN₄₋₈) . The zinc is removed by mild treatment with 1M HCl and recovered by chromatography on alumina. Metals can be inserted into this H₂(TPPF₂₀β-CN₄₋₈) by treatment with the metal salt in DMF, e.g., FeCl₂ in DMF, leading to Fe(TPPF₂₀β-CN₄₋₈)Cl.

### EXAMPLE 2

If the CuCN treatment is conducted under milder conditions some of the bromine groups can be retained leading to mixed bromo/cyano metalloporphyrins. Pyrrolic positions without cyano or bromo substitution can also be brominated, chlorinated or fluorinated leading to complexes of the general structure: 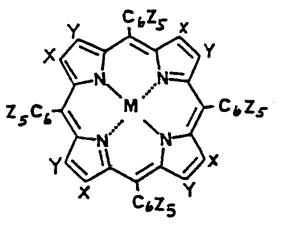
where
M is Fe, Cr, Mn, Ru, Co or Cu
X is CN
Y is CN, or Cl or Br or F
Z is H or Cl or F

### EXAMPLE 3

The conversion to cyano derivatives as disclosed in the examples above can also be applied to meso-perfluorinatedalkylporphyrins as disclosed in U.S. Patent Application Serial No. 568,118 filed August 16, 1990, (=EP-A-0 471 561). The general structure of the products is: 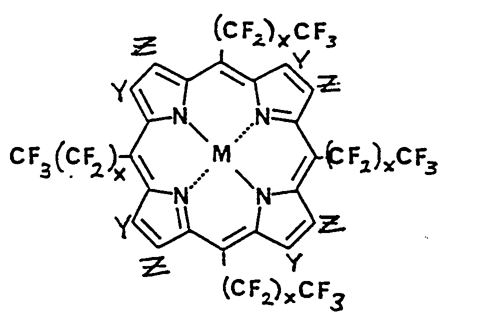
where
M is Fe, Cr, Mn, Ru, Cu, Co
X is 0-6
Y is CN
Z is CN or Cl or Br or F
The invention will be further disclosed with reference to the following example.

### EXAMPLE 4

The catalyst prepared as described in Example 1 is used as a catalyst for the oxidation of isobutane to t-butyl alcohol in the following manner. Isobutane (6-7 grams) is dissolved in 25 ml benzene containing the catalyst, and air is added to the desired pressure. Oxidation is carried out at the designated temperature for six hours. Gaseous and liquid products are analyzed by gas chromatography and mass spectrometry. Catalyst activity is expressed as "catalyst turnovers", i.e., moles of oxygen consumed/mole of catalyst. Selectivity is the moles of TBA per 100 moles of liquid product. Higher numbers of catalyst turnovers and/or greater selectivity are obtained with the catalyst of the invention as compared with otherwise similar catalyst which has not been substituted with cyano groups. Similar results are obtained when the catalysts of Examples 2 and 3 above are used as alkane oxidation catalysts.

### NEW COMPOUNDS

In this embodiment, the invention relates to metalloporphyrins useful as catalysts for the oxidation of alkanes, and more particularly to metalloporphyrins containing cyano groups on the porphyrin ring.

Cyano-substituted metalloporphyrins are known in the art, H. J. Callot, "Bromation de la m-tetraphenylporphine. Preparation d'alkyl - et de polycyanoporphines (1), Bull. soc. chim. de France 1974, No. 7-8, pages 1492-1496, discloses copper complexes of meso-tetraphenylporphyrins having cyano substituents on one, two, three and four of the pyrrolic rings. R. J. Donohoe, M. Atamian and D. F. Boclan, "Characterization of Singly Reduced Iron (II) Porphyrins", J. Am. Chem. Soc., 1987, 109, 5593-5599, disclose Fe(II)2,7,12-tricyano-5,10,15,20-tetraphenylporphyrin and Fe(II)2,7,12,17-tetracyano-5,10,15,20-tetraphenylporphyrin.

### DESCRIPTION OF THE INVENTION

We have discovered novel cyano-substituted metalloporphyrins which contain cyano groups in meso and/or beta positions of the porphyrin ring.

The atoms or groups on the meso positions of a metalloporphyrin are represented by the X's in the following structural formula, and the atoms or groups on the β-pyrrolic, or beta, positions by the Y's: 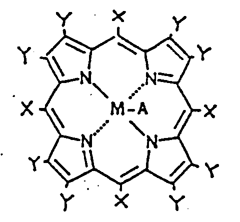
where M is metal, A (1) is an anion such as chloride, bromide, fluoride, cyanide, azide, nitride, thiocyanate, cyanate, hydroxy, methoxy, chlorate, carboxylates such as acetate, propionate and benzoate, or (2) is absent, which compounds include iron complexes of µ oxo dimers wherein two structures as shown in said formula are joined through an M-O-M linkage.

### METAL COMPLEXES OF MESOCYANOPORPHYRINS

In one embodiment of the invention, the metalloporphyrin has one or more cyano groups in meso positions and has, in beta positions, either hydrogen atoms, H, or halogen atoms such as fluorine, chlorine or bromine, or nitro or cyano groups, or a hydrocarbon group or a halocarbon group. Examples of halocarbon groups are haloalkyl groups such as perfluoromethyl, perfluoroethyl and the like and haloaryl groups such as perfluorophenyl. Examples of hydrocarbon groups are aryl groups such as phenyl and substituted phenyl, and alkyl or cycloalkyl groups such as methyl, ethyl and cyclohexyl.

In this embodiment, 1 to 4 of the X's in the above formula are CN, 0 to 3 of said X's are hydrogen, halogen, hydrocarbon or halocarbon, and Y is hydrogen, nitro, cyano, halogen, hydrocarbon or halocarbon. The Y's may all be one atom or group, or different atoms or groups

This embodiment differs from the meso-tetraphenyl cyanoporphyrins of the prior art in having cyano groups in meso positions of the metalloporphyrin complex.

### METAL COMPLEXES OF BETACYANOPORPHYRINS

In another embodiment of the invention, the metalloporphyrin has one or more cyano groups in beta positions, and hydrogen or a substituent other than cyano in the remaining beta positions. The substituent may be halo, hydrocarbon or halocarbon. In this embodiment, X in the above formula is hydrogen, halogen, nitro, cyano, alkyl, cycloalkyl or halocarbon, at least one of said Y's is cyano and the remaining Y's are hydrogen, halogen, nitro, hydrocarbon or halocarbon.

This embodiment differs from the meso-tetraphenylbetacyanoporphyrins of the prior art in having different substituents in the meso positions.

In a preferred embodiment, the compound has either halogen atoms or cyano groups in all of the beta positions. In this embodiment, X in the above formula is hydrogen, halogen, nitro, cyano, hydrocarbon or halocarbon, at least one of the Y's is cyano, and the remaining Y's are halogen.

This embodiment differs from the metal complexes of mesotetraphenylbetatetracyanoporphyrin of the prior art in having halogen substituents in beta positions.

Substituents in the meso positions of the metalloporphyrins of this embodiment may be aryl groups such as phenyl, or they may advantageously be perhalocarbon groups such as perfluoromethyl, perfluoroethyl and the like. In this embodiment, X in the above formula is a perhalocarbon group, and Y is hydrogen or CN, at least one of the Y's being CN.

This embodiment differs from the metal complexes of mesotetraphenylcyanoporphyrins of the prior art in having perhalocarbon groups in meso positions of the porphyrin ring.

In each embodiment of the invention, M in the above formula is preferably Fe, Cr, Mn, Ru, Cu or Co, more preferably Fe.

The compounds of the invention are useful for example as catalysts in the oxidation of organic compounds. The manner of usage of the compounds for this purpose is disclosed in applicants' copending application Serial No. 07/758148, filed September 12, 1991, issued June 2, 1992 as Patent No. 5,118,886.

The terms porphyrin, porphin and porphine are used interchangeably herein to refer to the structure shown in the structural formula supra.

The following examples illustrate the invention:

### Example 1

### Preparation of H₂P(CN)₄ and MP(CN)₄Cl

### P = Porphine

ZnP(NO₂)₄, zinc meso-tetranitroporphine (100 mg), is dissolved in 100 ml of glacial acetic acid. At 90-100°C, 0.5 g of KCN dissolved in 50 ml of tetrahydrofuran (THF) is dripped into the solution with stirring. The reaction is heated until TLC examination shows all of the starting material has reacted. The material is cooled, filtered and evaporated to dryness. The solid residue is washed thoroughly with water to remove excess KCN then dried and recrystallized from hot dichloromethane or chloroform/hexane. The zinc is removed during this process, leaving H₂P(CN)₄, meso-tetracyano-porphine. The metal salts of this complex are prepared by refluxing a THF solution of the H₂P(CN)₄ with the metal chloride (MCl₂.xH₂O where M is Fe, Cr, Co, Mn or Ru) until the metal is inserted and purifying by either recrystallization or chromatography.

### Example 2

### Preparation of Zn TPPF₂₀ β-Br₇CN and ZnTPPF₂₀ β-Br₆(CN)₂

### (TPP=tetraphenylporphine)

100 mg of ZnTPPF₂₀ β-Br₈ is dissolved in 90 ml of dimethylformamide. To this is added 129 mg of CuCN dissolved in 4.5 g of pyridine. The solution is stirred and refluxed for 5 hours then added to a saturated KCN solution. The porphyrin content is extracted with CH₂Cl₂ and evaporated to dryness in vacuo at 90°C to remove the pyridine in addition to the CH₂Cl₂. After chromatography on silica gel two major bands are obtained other than some starting material. The first green band elutes from the column with CHCl₃. Both bands have infrared (KBr) _{C-N} around 2220 cm⁻¹. The first green material is identified as ZnTPPF₂₀ β-Br₇CN and the second brown band as a mixture of isomers of ZnTPPF₂₀ β-Br₆(CN)₂.

The zinc is removed by treating a CH₂Cl₂ solution of either of the cyano porphyrins with a few bubbles of HCl gas at room temperature followed by neutralization with bicarbonate solution. Complexes of metals such as Fe, Cr, Mn, Cu and Ru can be obtained by stirring the metal chloride (FeCl₂, CrCl₂, CoCl₂), or carbonyl (Ru₃(CO)₁₂) with the free porphyrin in hot dimethylformamide.

## Claims

1. Process for oxidation of alkanes which comprises contacting alkane with oxygen-containing gas in the presence of metalloporphyrin in which hydrogen atoms in the porphyrin ring have been substituted with at least one cyano group.

2. Process according to claim 1 in which 1 to 8 of the pyrrolic hydrogens have been replaced with cyano groups.

3. Process according to claim 1 wherein the metalloporphyrin contains iron, chromium, manganese, ruthenium, cobalt or copper.

4. Process according to claim 2 in which remaining hydrogen atoms in the porphyrin ring have been replaced by halogens.

5. Process according to claim 4 in which 4 to 8 hydrogen atoms in the porphyrin ring have been replaced with cyano groups and in which 8 to 20 hydrogen atoms in the porphyrin ring have been replaced with halogen.

6. Process according to claim 1 in which the metalloporphyrin is a cyanated, meso-perfluorinated alkyl porphyrin.

7. Process according to claim 1 in which the metalloporphyrin is a cyanated iron tetrakis pentafluorophenylporphyrin.

8. Process according to claim 1 wherein the metalloporphyrin is a metallo mesotetracyanoporphine.

9. A compound having the formula: 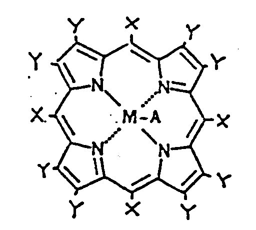 where M is iron, chromium, manganese, ruthenium, copper or cobalt, 1 to 4 of said X's are cyano, 0 to 3 of said X's are hydrogen, halogen, hydrocarbon or halocarbon, Y is hydrogen, halogen, nitro, cyano, hydrocarbon or halocarbon, and A is an anion such as chloride, bromide, fluoride, cyanide, azide, nitride, thiocyanate, cyanate, hydroxy, methoxy, chlorate, carboylate, or is absent, said compounds including iron complexes of µ oxo dimers comprising two structures as shown in said formula joined through an M-O-M linkage.

10. Compound according to claim 9 wherein each X is cyano.

11. Compound according to claim 10 wherein M is iron and each Y is hydrogen.

12. Compound according to claim 9, which is a metal complex of mesotetracyanoporphyrin or mesotetracyanoporphyrin halide, where the metal is iron, chromium, manganese, ruthenium, copper or cobalt.

13. Compound according to claim 9 wherein A is chloride, bromide, fluoride, hydroxy or azide.

14. Compound according to claim 9 wherein said compound is said iron complex of µ oxo dimer.

15. A compound having the formula: 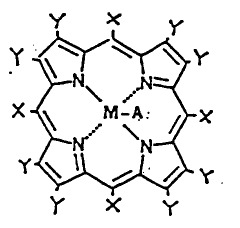 where M is iron, chromium, manganese, ruthenium, copper or cobalt, X is hydrogen, halogen, nitro, cyano, alkyl, cycloalkyl or halocarbon, at least one of said Y's is cyano the remaining Y's are hydrogen, halogen, nitro, cyano, hydrocarbon or halocarbon, A is an anion such as chloride, bromide, fluoride, cyanide, azide, nitride, thiocyanate, cyanate, hydroxy, methoxy, chlorate, carboxylate, or is absent, said compounds including iron complexes of µ oxo dimers comprising two structures as shown in said formula joined through an M-O-M linkage.

16. Compound according to claim 15 wherein said remaining Y's are halogen.

17. Compound according to claim 15 wherein each X is fluorocarbon.

18. Compound according to claim 15, which is an iron complex of mesotetrafluoroalkylbetacyanoporphyrin or mesotetrafluoroalkylbeta cyanoporphyrin halide, having 1 to 7 carbon atoms in said alkyl group.

19. Compound according to claim 15, which is a metal complex of mesotetraphenyl β-heptabromo β-cyanoporphine, where said metal is iron, chromium, manganese, ruthenium, copper or cobalt.

20. Compound according to claim 15, which is a metal complex of mesotetraphenyl β-hexabromo β-dicyanoporphine, where said metal is iron, chromium, ruthenium, copper or cobalt.

21. Compound according to claim 15 wherein said compound is said iron complex of µ oxo dimer.

## Patentansprüche

1. Verfahren zur Oxidation von Alkanen, welches das Kontaktieren eines Alkans mit sauerstoffhaltigem Gas in Gegenwart eines Metallporphyrins, in welchem Wasserstoffatome im Porphyrinring durch mindestens eine Cyangruppe ersetzt worden sind, umfaßt.

2. Verfahren gemäß Anspruch 1, wobei 1 bis 8 der Pyrrolwasserstoffatome durch Cyangruppen ersetzt worden sind.

3. Verfahren gemäß Anspruch 1, wobei das Metallporphyrin Eisen, Chrom, Mangan, Ruthenium, Kobalt oder Kupfer enthält.

4. Verfahren gemäß Anspruch 2, wobei die verbleibenden Wasserstoffatome im Porphyrinring durch Halogenatome ersetzt worden sind.

5. Verfahren gemäß Anspruch 4, wobei 4 bis 8 Wasserstoffatome im Porphyrinring durch Cyangruppen ersetzt worden sind und wobei 8 bis 20 Wasserstoffatome im Porphyrinring durch Halogenatome ersetzt worden sind.

6. Verfahren gemäß Anspruch 1, wobei das Metallporphyrin ein cyaniertes, mesoperfluoriertes Alkylporphyrin ist.

7. Verfahren gemäß Anspruch 1, wobei das Metallporphyrin ein cyaniertes Eisentetrakispentafluorphenylporphyrin ist.

8. Verfahren gemäß Anspruch 1, wobei das Metallporphyrin ein Metallmesotetracyanoporphin ist.

9. Verbindung der Formel: 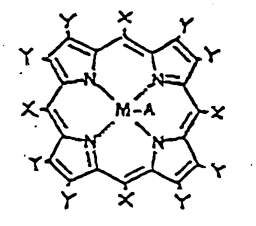 worin M Eisen, Chrom, Mangan, Ruthenium, Kupfer oder Cobalt ist, 1 bis 4 der Substituenten X Cyangruppen sind, 0 bis 3 der Substituenten X Wasserstoff, Halogen, Kohlenwasserstoff oder Halogenkohlenstoff sind, Y Wasserstoff, Halogen, Nitro, Cyan, Kohlenwasserstoff oder Halogenkohlenstoff ist und A ein Anion, zum Beispiel Chlorid, Bromid, Fluorid, Cyanid, Azid, Nitrid, Thiocyanat, Cyanat, Hydroxy, Methoxy, Chlorat oder Carboxylat, ist oder nicht vorhanden ist, wobei die Verbindung Eisenkomplexe aus µ-Oxodimeren umfaßt, welche zwei der in der Formel gezeigten Strukturen enthalten, die durch eine M-O-M Bindung verknüpft sind.

10. Verbindung gemäß Anspruch 9, worin jedes X eine Cyangruppe ist.

11. Verbindung gemäß Anspruch 10, worin M Eisen und jedes Y Wasserstoff ist.

12. Verbindung gemäß Anspruch 9, die ein Metallkomplex aus Mesotetracyanoporphyrin oder Mesotetracyanoporphyrinhalogenid ist, wobei das Metall Eisen, Chrom, Mangan, Ruthenium, Kupfer oder Cobalt ist.

13. Verbindung gemäß Anspruch 9, worin A Chlorid, Bromid, Fluorid, Hydroxy oder Azid ist.

14. Verbindung gemäß Anspruch 9, worin die Verbindung der Eisenkomplex eines µ-Oxodimeren ist.

15. Verbindung der Formel: 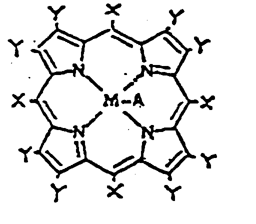 worin M Eisen, Chrom, Mangan, Ruthenium, Kupfer oder Cobalt ist, X Wasserstoff, Halogen, Nitro, Cyan, Alkyl, Cycloalkyl oder Halogenkohlenstoff ist, mindestens ein Y Cyan ist, wobei die übrigen Substituenten Y Wasserstoff, Halogen, Nitro, Cyan, Kohlenwasserstoff oder Halogenkohlenstoff sind, A ein Anion, zum Beispiel Chlorid, Bromid, Fluorid, Cyanid, Azid, Nitrid, Thiocyanat, Cyanat, Hydroxy, Methoxy, Chlorat oder Carboxylat, ist oder nicht vorhanden ist, wobei die Verbindung Eisenkomplexe aus µ-Oxodimeren umfaßt, welche zwei der in der Formel gezeigten Strukturen enthalten, die durch eine M-O-M Bindung verknüpft sind.

16. Verbindung gemäß Anspruch 15, worin die übrigen Substituenten Y Halogen sind.

17. Verbindung gemäß Anspruch 15, worin jedes X Fluorkohlenstoff ist.

18. Verbindung gemäß Anspruch 15, die ein Eisenkomplex aus Mesotetrafluoralkyl-β-cyanoporphyrin oder Mesotetrafluoralkyl-β-cyanoporphyrinhalogenid ist, wobei die Alkylgruppe 1 bis 7 Kohlenstoffatome aufweist.

19. Verbindung gemäß Anspruch 15, die ein Metallkomplex aus Mesotetraphenyl-β-heptabromo-β-cyanoporphin ist, worin das Metall Eisen, Chrom, Mangan, Ruthenium, Kupfer oder Cobalt ist.

20. Verbindung gemäß Anspruch 15, die ein Metallkomplex aus Mesotetraphenyl-β-hexabromo-β-dicyanporphin ist, wobei das Metall Eisen, Chrom, Ruthenium oder Cobalt ist.

21. Verbindung gemäß Anspruch 15, wobei die Verbindung der Eisenkomplex eines µ-Oxodimeren ist.

## Revendications

1. Procédé d'oxydation d'alcanes, caractérisé en ce que l'on met un alcane en contact avec un gaz contenant de l'oxygène, en présence d'une métalloporphyrine dans laquelle des atomes d'hydrogène du noyau porphyrine ont été substitués par au moins un groupe cyano.

2. Procédé suivant la revendication 1, caractérisé en ce que de 1 à 8 des hydrogènes pyrroliques ont été remplacés par des radicaux cyano.

3. Procédé suivant la revendication 1, caractérisé en ce que la métalloporphyrine contient du fer, du chrome, du manganèse, du ruthénium, du cobalt ou du cuivre.

4. Procédé suivant la revendication 2, caractérisé en ce que les atomes d'hydrogène résiduels du noyau porphyrine ont été remplacés par des atomes d'halogènes.

5. Procédé suivant la revendication 4, caractérisé en ce que de 4 à 8 atomes d'hydrogène du noyau porphyrine ont été remplacés par des groupes cyano et en ce que 8 à 20 atomes d'hydrogène du noyau porphyrine ont été remplacés par des atomes d'halogènes.

6. Procédé suivant la revendication 1, caractérisé en ce que la métalloporphyrine est une alkylporphyrine cyanatée, mésoperfluorée.

7. Procédé suivant la revendication 1, caractérisé en ce que la métalloporphyrine est une tétrakispentafluorophénylporphyrine de fer, cyanatée.

8. Procédé suivant la revendication 1, caractérisé en ce que la métalloporphyrine est une métallomésotétracyanoporphine.

9. Composé répondant à la formule suivante : 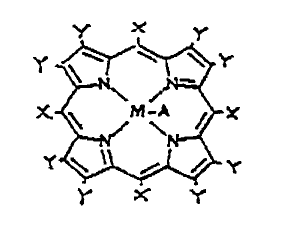 dans laquelle M représente le fer, le chrome, le manganèse, le ruthénium, le cuivre ou le cobalt, de 1 à 4 des symboles X représentent des radicaux cyano, de 0 à 3 des symboles X représentent des atomes d'hydrogène, des atomes d'halogènes, des restes d'hydrocarbures ou d'hydrocarbures halogénés, Y représente un atome d'hydrogène, un atome d'halogène, un radical nitro, cyano, un reste d'hydrocarbure ou d'hydrocarbure halogéné, et A est un anion tel qu'un anion chlorure, bromure, fluorure, cyanure, azide, nitrure, thiocyanate, cyanate, hydroxyle, méthoxy, chlorate, carboxylate, ou est absent, ledit composé comprenant des complexes du fer de µ-oxodimères comprenant deux structures telles que représentées dans ladite formule, unies par une liaison M-O-M.

10. Composé suivant la revendication 9, caractérisé en ce que chaque symbole X représente un radical cyano.

11. Composé suivant la revendication 10, caractérisé en ce que M représente le fer et chaque symbole Y représente un atome d'hydrogène.

12. Composé suivant la revendication 9, caractérisé en ce qu'il est un complexe d'un métal de la mésotétracyanoporphyrine ou d'un halogénure de mésotétracyanoporphyrine, où le métal est le fer, le chrome, le manganèse, le ruthénium, le cuivre ou le cobalt.

13. Composé suivant la revendication 9, caractérisé en ce que A représente un anion chlorure, bromure, fluorure, hydroxyle ou azide.

14. Composé suivant la revendication 9, caractérisé en ce qu'il est un complexe du fer d'un µ-oxodimère.

15. Composé répondant à la formule suivante : 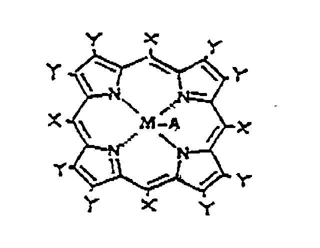 dans laquelle M est le fer, le chrome, le manganèse, le ruthénium, le cuivre ou le cobalt, X représente un atome d'hydrogène, un atome d'halogène, un radical nitro, cyano, alkyle, cycloalkyle ou un reste d'hydrocarbure halogéné, au moins l'un des symboles Y représente un radical cyano, les symboles Y résiduels représentent des atomes d'hydrogène, des atomes d'halogènes, des radicaux nitro, cyano, des restes d'hydrocarbures ou d'hydrocarbures halogénés, A est un anion, tel qu'un anion chlorure, bromure, fluorure, cyanure, azide, nitrure, thiocyanate, cyanate, hydroxyle, méthoxy, chlorate, carboxylate, ou est absent, le composé comprenant des complexes du fer de µ-oxodimères comprenant deux structures telles que représentées dans ladite formule, unies par l'intermédiaire d'une liaison M-O-M.

16. Composé suivant la revendication 15, caractérisé en ce que les symboles Y résiduels représentent des atomes d'halogènes.

17. Composé suivant la revendication 15, caractérisé en ce que chaque symbole X représente un reste d'hydrocarbure fluoré.

18. Composé suivant la revendication 15, caractérisé en ce qu'il est un complexe du fer d'une mésotétrafluoralkyl-β-cyanoporphyrine, ou d'un halogénure de mésotétrafluoralkyl-β-cyanoporphyrine, dont le radical alkyle comporte de 1 à 7 atomes de carbone.

19. Composé suivant la revendication 15, caractérisé en ce qu'il est un complexe d'un métal de la mésotétraphényl-β-heptabromo-β-cyanoporphine, où le métal est le fer, le chrome, le manganèse, le ruthénium, le cuivre ou le cobalt.

20. Composé suivant la revendication 15, caractérisé en ce qu'il est un complexe d'un métal de la mésotétraphényl-β-hexabromo-β-dicyanoporphine, où le métal est le fer, le chrome, le ruthénium, le cuivre ou le cobalt.

21. Composé suivant la revendication 15, caractérisé en ce qu'il est un complexe du fer d'un µ-oxodimère.
